# EUROPEAN PATENT APPLICATION

(11) **EP 4 530 292 A1**
(43) Date of publication of application: **02.04.2025**
(21) Application number: 23812103.2
(22) Date of filing: 23.05.2023
(51) Int. Cl.: C07K 14/005, C07K 14/35, C12N 15/70, C12N 15/62

(54) **CRYSTALLINE-PROTEIN-BASED PLATFORM USING VIRAL NUCLEOCAPSID FOR PRODUCING TARGET-PROTEIN-FUSED SELF-ASSEMBLED NANOPARTICLES**

(30) Priority: 23.05.2022 KR 20220062790
(71) Applicant: Vaxdigm Co., Ltd., Seoul 06097 (KR); University-Industry Foundation, Yonsei University, Seoul 03722 (KR)
(72) Inventor: SOHN, Myung Hyun, Seoul 02878 (KR); PARK, Seong Hyun, Incheon 21093 (KR); HAN, Jae Won, Incheon 21986 (KR); KIM, Sung Jae, Seoul 06337 (KR); KIM, Seung Hoo, Seoul 02145 (KR)
(74) Representative: Algemeen Octrooi- en Merkenbureau B.V.
(86) International application number: PCT/KR2023/006959
(87) International publication number: WO 2023/229329

(57) **Abstract**

An expression vector for producing target protein-fused self-assembled nanoparticles of the present invention includes a polynucleotide that encodes a crystallizable protein and a viral nucleocapsid as fusion partners of the target protein. In addition, a method of producing target protein-fused self-assembled nanoparticles of the present invention includes manufacturing an expression vector for producing target protein-fused self-assembled nanoparticles, including a polynucleotide that encodes a target protein and a crystallizable protein and a viral nucleocapsid as fusion partners of the target protein; producing a transformant by introducing the expression vector for producing target protein-fused self-assembled nanoparticles into host cells; and culturing the transformant.

## Description

### [Technical Field]

The present invention relates to a platform for producing target protein-fused self-assembled nanoparticles based on a crystallizable protein using a viral nucleocapsid. More particularly, the present invention relates to an expression vector which can efficiently produce self-assembled nanoparticles, which are fused with a target protein, particularly, a difficult-to-express protein (DtEP) expressed insoluble in *E. coli,* and present the target protein on the surface, for example, in an immunologically meaningful structure, in host cells, especially, *E. coli* using a crystallizable protein and a viral nucleocapsid as fusion partners, a host cell transformed with the expression vector, and a method of producing target protein-fused self-assembled nanoparticles using the same.

### [Background Art]

Among vaccine technologies for coping with infectious diseases, recently, a chimeric VLP technology for introducing a foreign pathogenic antigen to a virus-like particle (VLP) structure which forms a polymer structure well was developed, and has been used to develop vaccines targeting various pathogens. This chimeric VLP presents a foreign pathogenic antigen on the surface of a VLP-based structure composed of a structural protein or self-assembled protein of another specific virus.

A chemical conjugation or genetic fusion method is used to introduce a foreign antigen into the VLP-based structure. The chemical conjugation method chemically fuses a separately prepared foreign antigen to the VLP-based structure that was already prepared through covalent bonding, but has disadvantages of difficult arrangement of the antigens in a certain direction and relatively low density of the presented antigens. On the other hand, the genetic fusion method has the advantage that chimeric VLPs can be prepared through a simpler process by fusion-expressing a gene of a VLP structure-forming protein and a gene of a target antigen protein at the DNA level. However, this method has the problem of limitations in foreign antigens that can be introduced, and particularly, of abnormal protein expression due to the absence of post-translational chaperones or processing, for example, the formation of an insoluble aggregate of proteins, when an *E. coli* expression system is used for highly-efficient rapid production. Therefore, it is necessary to develop technology to resolve the above problems.

### [Disclosure]

### [Technical Problem]

The present invention is directed to providing an expression vector that can efficiently produce self-assembled nanoparticles, which are fused with a target protein, particularly, a difficult-to-express protein (DtEP) expressed insoluble in *E. coli,* and present the target protein on the surface, for example, in an immunologically meaningful structure in host cells, especially, *E. coli.*

The present invention is also directed to providing a host cell which is transformed with the expression vector to efficiently produce the self-assembled nanoparticles.

The present invention is also directed to providing a method of efficiently producing the self-assembled nanoparticles using the expression vector and/or the host cells.

### [Technical Solution]

The present invention provides an expression vector for producing target protein-fused self-assembled nanoparticles, which includes a polynucleotide encoding a crystallizable protein and a viral nucleocapsid as fusion partners of a target protein.

The present invention also provides a host cell, which is transformed with the expression vector for producing target protein-fused self-assembled nanoparticles.

The present invention also provides a method of producing target protein-fused self-assembled nanoparticles, which includes manufacturing an expression vector for producing target protein-fused self-assembled nanoparticles, including a polynucleotide that encodes a target protein, and a crystallizable protein and a viral nucleocapsid as fusion partners of the target protein; producing a transformant by introducing the expression vector for producing target protein-fused self-assembled nanoparticles into host cells; and culturing the transformant.

### [Advantageous Effects]

According to the present invention, self-assembled nanoparticles, which are fused with a target protein, particularly, a difficult-to-express protein (DtEP) expressed insoluble in *E. coli,* and present the target protein on the surface, for example, in an immunologically meaningful structure, can be efficiently produced in host cells, especially, *E. coli.*

### [Description of Drawings]

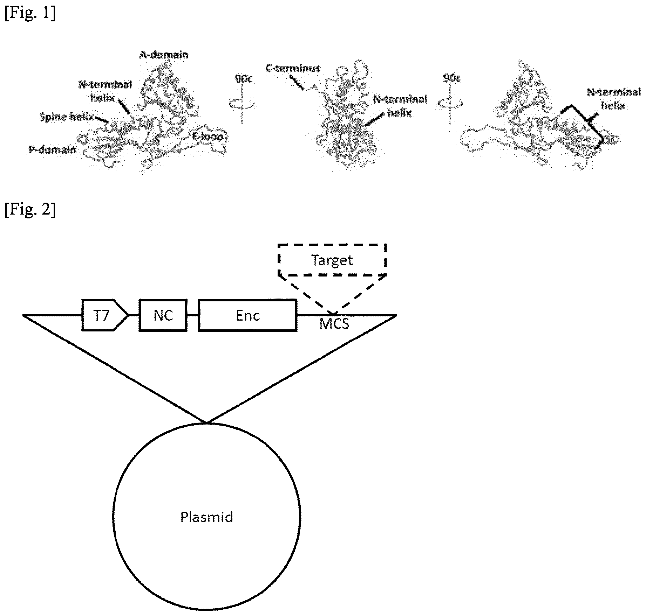

FIG. 1 shows the structure of *Mycobacterium tuberculosis-derived* encapsulin as an example of a crystallizable protein, encapsulin protein.
FIG. 2 shows an expression vector according to one embodiment of the present invention (T7, T7 promoter; NC, viral nucleocapsid-coding site; Enc, encapsulin-coding site; Target, target protein-coding site; MCS, multiple cloning site).
FIG. 3 shows the schematic concept of the present invention.
FIG. 4 shows a fusion protein construct for investigating the applicability of the combination of a crystallizable protein and a viral nucleocapsid (NC) as fusion partners for producing self-assembled nanoparticles.
FIGS. 5 and 6 show fusion protein construct regions in an expression vector according to one embodiment of the present invention, respectively.
FIG. 7 shows the SDS-PAGE results that confirm the fusion protein construct of FIG. 4 expressed in *E. coli.*
FIG. 8 shows the results of observing a product obtained by the expression of the fusion protein construct of FIG. 4 (self-assembled nanoparticles) through transmission electron microscopy (TEM).
FIG. 9 shows the results of analyzing the product obtained by the expression of the fusion protein construct of FIG. 4 (self-assembled nanoparticles) through size exclusion chromatography (SEC).
FIG. 10 shows the results of observing the product obtained by the expression of the fusion protein construct of FIG. 4 (self-assembled nanoparticles) through cryo-electron microscopy (Cryo-EM).
FIG. 11 shows the results of analyzing the locations of the N- and C-terminal regions of a fusion protein on the product obtained by the expression of the fusion protein construct of FIG. 4 (self-assembled nanoparticles).
FIG. 12 shows the SDS-PAGE results that confirm the fusion protein construct of FIG. 5 expressed in *E. coli.*
FIG. 13 shows the results of observing a product obtained by the expression of the fusion protein construct of FIG. 5 (self-assembled nanoparticles) through TEM.
FIG. 14 shows the results of investigating the functionality of the product obtained by the expression of the fusion protein construct of FIG. 5 (self-assembled nanoparticles) as an antigen.
FIG. 15 shows the SDS-PAGE results that confirm the fusion protein construct of FIG. 6 expressed in *E. coli.*
FIG. 16 shows the results of observing a product obtained by the expression of the fusion protein construct of FIG. 6 (self-assembled nanoparticles) through TEM.

### [Modes of the Invention]

According to one embodiment of the present invention, an expression vector for producing target protein-fused self-assembled nanoparticles, which includes a polynucleotide that encodes a crystallizable protein and a viral nucleocapsid as fusion partners of a target protein, is provided.

The term "target protein" used herein refers to a protein to be produced in large quantities by one of ordinary skill in the art, including all proteins that can be expressed in host cells after inserting a polynucleotide encoding the protein in a recombinant vector.

In the present invention, a target protein may be selected from the group consisting of an antigen, an antibody, a cell receptor, an enzyme, a structural protein, serum, and a cell protein, but the present invention is not limited thereto.

The term "expression vector" used herein is a vector for expressing a polynucleotide encoding a polypeptide of interest, which has been introduced into a heterologous or homologous host cell, and refers to a circular or linear polynucleotide, for example, a DNA or RNA molecule. An expression vector may include a promoter sequence and/or a terminator sequence, and may also include components required for use in a vector, such as a replication origin, a selection marker, a polyadenylation signal, etc.

The expression vector of the present invention may be a plasmid, a viral vector, a phage particle, or a genome insert, and may be replicated regardless of the genome of a host cell or integrated into the genome of a host cell after being introduced into the host cell.

The expression vector of the present invention may include a polynucleotide encoding a target protein, or may be used to include a polynucleotide encoding a target protein in the future. Particularly, in the latter case, as a component that allows easy inclusion of a target protein-encoding polynucleotide, a restriction enzyme-recognition site usually called a multiple cloning site (MSC) is preferably included.

The present invention is based on new research results: When a crystallizable protein and a viral nucleocapsid are applied as fusion partners for a target protein, the target protein can be efficiently expressed (for example, a protein that is expressed insoluble when no fusion partner is used or when another existing fusion partner is used can be expressed soluble); and when a crystallizable protein and a viral nucleocapsid are applied as fusion partners for a target protein, nanoparticles in which the target protein is self-assembled can be efficiently produced.

Particularly, the viral nucleocapsid used as a fusion partner in the present invention has the advantage of minimizing the interference with a target protein because the viral nucleocapsid has a very small size (short peptide). When using such a fusion partner, there is an advantage of producing a target protein with increased solubility, and in general, the larger the size of the fusion partner, the more helpful it is in enhancing the water solubility of the target protein. However, increased structural interference with the adjacent target protein due to the size of the fusion partner may also impede production of a physiologically active structure. Therefore, it is very important to discover a fusion partner that increases the solubility and activity of the target protein despite its small size, if possible. Particularly, in the present invention, the viral nucleocapsid used as a fusion partner has a very small size (short peptide), so it has the advantages of minimizing structural interference with the target protein and minimizing structural interference with the self-assembly of a monomer of the target protein (in the present invention, self-assembly into nanoparticles). In general, to avoid such structural interference, conventional technologies require the removal of the fusion partner by treating the fusion protein with a specific cleavage enzyme after production, and therefore, increased production costs due to the use of an expensive enzyme and additional cleavage, and a purification process remain as an obstacle. The present invention can technologically and dramatically improve this issue.

In addition, according to the present invention, there is the advantage of utilizing the zinc-binding domain of the viral nucleocapsid. For example, by utilizing the zinc-binding domain of the viral nucleocapsid, for example, a zinc affinity purification method, a fusion protein can be easily purified without separate components for purification such as a histidine tag.

In the present invention, the viral nucleocapsid may be a conventionally known viral nucleocapsid, and it may be a fragment or variant of the viral nucleocapsid, as long as the activity of the present invention is retained, in other words, the activity to form self-assembled nanoparticles in which the viral nucleocapsid acts as a fusion partner for a target protein along with a crystallizable protein to allow the target protein to be expressed soluble especially in *E. coli* and present on the surface, particularly, as an immunologically meaningful structure is retained.

In the present invention, the viral nucleocapsid may be a nucleocapsid of a retrovirus, for example, human immunodeficiency virus-1 (HIV-1), SIV, or MuLV.

In the present invention, the viral nucleocapsid is preferably a nucleocapsid of human immunodeficiency virus (HIV).

In the present invention, the viral nucleocapsid may include an amino acid sequence having 50% or more, 60% or more, 70% or more, 80% or more, 90% or more, 95% or more, 96% or more, 97% or more, 98% or more, or 99% or more sequence identity with the amino acid sequence of SEQ ID NO: 1, or the amino acid sequence of SEQ ID NO: 1. The viral nucleocapsid includes, preferably, an amino acid sequence which retains amino acids 14, 17, 22, and 27, more preferably, amino acids 14, 17, 22, 27, 35, 38, 43, and 48, and even more preferably, amino acids 6, 9, 10, 13, 14, 17, 19, 22, 25, 27, 28, 31, 32, 33, 35, 37, 38, 40, 43, 46, 48, and 51 of SEQ ID NO: 1, and satisfies the above-mentioned sequence identity.

In the present invention, the polynucleotide encoding the viral nucleocapsid includes a nucleotide sequence having, preferably, 50% or more, 60% or more, 70% or more, 80% or more, 90% or more, 95% or more, 96% or more, 97% or more, 98% or more, or 99% or more sequence identity with the nucleotide sequence of SEQ ID NO: 2 or 3, or the nucleotide sequence of SEQ ID NO: 2 or 3. The polynucleotide encoding the viral nucleocapsid includes a nucleotide sequence that encodes an amino acid sequence that retains, preferably, amino acids 14, 17, 22, and 27, more preferably, amino acids 14, 17, 22, 27, 35, 38, 43, and 48, and even more preferably, amino acids 6, 9, 10, 13, 14, 17, 19, 22, 25, 27, 28, 31, 32, 33, 35, 37, 38, 40, 43, 46, 48, and 51 of SEQ ID NO: 1, and satisfies the above-mentioned sequence identity.

The sequence identity used herein means the sequence identity between an amino acid sequence and a reference amino acid sequence, or between a nucleotide sequence and a reference nucleotide sequence. The sequence identity may be determined by comparing the positions in sequences that can be aligned for comparison. When a position in the comparative sequence is occupied by the same amino acid or base, molecules are identical at the position. The degree of identity between amino acid or nucleotide sequences is a function of the number of identical amino acids or nucleotides at each position shared by the same amino acid or nucleotide sequence. For example, using a variety of alignment algorithms and/or programs, including FASTA or BLAST, the identity between two sequences may be calculated.

In the present invention, the crystallizable protein may be used without limitation as long as each protein is a protein with the activity to form an assembly, for example, a cage-shaped complex protein as a unit. For example, as the crystallizable protein, encapsulin or ferritin may be used.

In the present invention, conventionally known encapsulin or ferritin as well as a fragment of encapsulin or ferritin may be used as long as the activity to form an assembly, for example, a cage-shaped complex protein as a unit, is maintained.

Encapsulin derived from an organism such as *Mycobacterium tuberculosis, Thermotoga maritima, Pyrococcus furiosus,* or *Myxococcus xanthus* may be used, and preferably, *Mycobacterium tuberculosis-derived* encapsulin is used.

In the present invention, the crystallizable protein preferably includes an amino acid sequence having 50% or more, 60% or more, 70% or more, 80% or more, 90% or more, 95% or more, 96% or more, 97% or more, 98% or more, or 99% or more sequence identity with the amino acid sequence of SEQ ID NO: 4 or 6, or the amino acid sequence of SEQ ID NO: 4 or 6.

In the present invention, the polynucleotide encoding the crystallizable protein preferably includes a nucleotide sequence having 50% or more, 60% or more, 70% or more, 80% or more, 90% or more, 95% or more, 96% or more, 97% or more, 98% or more, or 99% or more sequence identity with the nucleotide sequence of SEQ ID NO: 5 or 7, or the nucleotide sequence of SEQ ID NO: 5 or 7.

The expression vector of the present invention may be for producing target protein-fused self-assembled nanoparticles in cells selected from all organisms including prokaryotes and eukaryotes, preferably, a microorganism of the genus *Escherichia,* and more preferably, *E. coli.*

In the expression vector of the present invention, the polynucleotide encoding a crystallizable protein and a viral nucleocapsid may further include other components, for example, a cloning site for inserting a target protein-encoding polynucleotide, such as a restriction enzyme recognition site, e.g., a multiple cloning site (MCS); a linker sequence for providing a gap between a target protein and a fusion partner and/or between fusion partners (between a crystallizable protein and a viral nucleocapsid); a tag sequence such as a histidine-tagging sequence; and/or a protease recognition site-encoding sequence for cleaving a portion of an expressed fusion protein. Each component may be arranged in various ways, if needed, or the number of these components may vary.

In one embodiment, the expression vector of the present invention includes a polynucleotide that encodes a crystallizable protein and a viral nucleocapsid as fusion partners of a target protein, and the polynucleotide encodes a crystallizable protein and a viral nucleocapsid linked to the N-terminus of the crystallizable protein, and includes a target protein-coding site or a cloning site for linking a target protein to the C-terminus of the crystallizable protein. According to this form, self-assembled nanoparticles may be produced in such a way that the target protein is exposed on the outside of the nanoparticle and the viral nucleocapsid is located inside the nanoparticle, which is particularly effective in producing antigen-presenting nanoparticles for an immune response. As a specific example of the embodiment, the polynucleotide includes a construct in which a promoter, a viral nucleocapsid-coding site, a crystallizable protein-coding site, a target protein-coding site or a cloning site for inserting a target protein-coding polynucleotide (e.g., a restriction enzyme recognition site, e.g., MCS), and a terminator are operably linked sequentially in the 5' to 3' direction.

In another embodiment, the expression vector of the present invention includes a polynucleotide that encodes a crystallizable protein and a viral nucleocapsid as fusion partners of the target protein, and the polynucleotide encodes a crystallizable protein and a viral nucleocapsid linked to the N-terminus of the crystallizable protein, and includes a target protein-coding site or a cloning site for linking a target protein between the crystallizable protein and the viral nucleocapsid. As a specific example of the embodiment, the polynucleotide includes a construct in which a promoter, a virus nucleocapsid-coding site, a target protein-coding site or a cloning site for inserting a target protein-coding polynucleotide (e.g., a restriction enzyme recognition site, e.g., MCS), a crystallizable protein-coding site and a terminator are operably linked sequentially in the 5' to 3' direction.

In still another embodiment, the expression vector of the present invention includes a polynucleotide that encodes a crystallizable protein and a viral nucleocapsid as fusion partners of the target protein, and the polynucleotide encodes a crystallizable protein and a viral nucleocapsid linked to the N-terminus of the crystallizable protein, and includes cloning sites for linking a target protein at the C-terminus of the crystallizable protein and between the crystallizable protein and the viral nucleocapsid. In this case, a user may choose whether to position the target protein at the C-terminus or the N-terminus of the crystallizable protein. As a specific example of the embodiment, the polynucleotide includes a construct in which a promoter, a virus nucleocapsid-coding site, a first cloning site for inserting the target protein-coding polynucleotide (e.g., a restriction enzyme recognition site, e.g., MCS), a crystallizable protein-coding site, a second cloning site for inserting the target protein-coding polynucleotide (e.g., a restriction enzyme recognition site, e.g., MCS), and a terminator are operably linked sequentially in the 5' to 3' direction.

The expression vector of the present invention may include a T7 promoter and a T7 terminator to regulate the transcription of the target protein and fusion partner-encoding DNA by a bacteriophage T7 system. According to this, the expression vector of the present invention can more effectively produce the target protein in *E*. *coli.*

The expression vector of the present invention may be manufactured based on, for example, a conventional expression vector using a conventional recombination method.

According to yet another embodiment of the present invention, a host cell transformed with an expression vector for producing target protein-fused self-assembled nanoparticles of the present invention is provided.

The host cell in the present invention may be selected from cells of all organisms including prokaryotes and eukaryotes, preferably, cells of a microorganism of the genus *Escherichia,* and more preferably, *E. coli.*

The term "transformation" or "introduction" used herein means introducing a polynucleotide into a host so that the polynucleotide becomes replicable either as an extrachromosomal element or by completion of chromosomal integration. A transformation method with the expression vector according to the present invention may include electroporation, a calcium phosphate (CaPO₄) method, a calcium chloride (CaCl₂) method, microinjection, a polyethylene glycol (PEG) method, a DEAE-dextran method, a cationic liposome method, or a lithium acetate-DMSO method, but the present invention is not limited thereto.

According to yet another embodiment of the present invention, as a method of efficiently producing target protein-fused self-assembled nanoparticles in heterologous or homologous host cells, a method of producing target protein-fused self-assembled nanoparticles, which includes manufacturing an expression vector for producing target protein-fused self-assembled nanoparticles, which includes a polynucleotide that encodes a target protein and a crystallizable protein and a viral nucleocapsid as fusion partners of the target protein; producing a transformant by introducing the expression vector for producing target protein-fused self-assembled nanoparticles into host cells; and culturing the transformant is provided.

Here, for specific details, such as matters regarding the expression vector and matters regarding the introduction of the expression vector, the previously described matters may be referred to.

For culturing a transformant, any culture method known to be suitable for a host cell may be used. For example, when the host cell is *E. coli,* a method of culturing in Luria-Bertani (LB) medium at 16 to 37 °C may be used. If there is a component of an antibiotic selection marker (e.g., a gene resistant to the antibiotic ampicillin) in the expression vector, at this time, the proliferation of *E. coli* containing the expression vector can be preferentially induced by adding the corresponding antibiotic (e.g., ampicillin) to the medium and culturing the cells. In addition, when the expression vector contains a component that regulates the transcription and/or translation of DNA encoding the target protein, for example, a lac operon-related component, in the expression vector, the expression of the target protein can be induced by adding a related material, such as IPTG.

The following examples are presented to help understand the present invention and the following contents do not limit the scope of the present invention.

### [Examples]

### Preparation Example 1

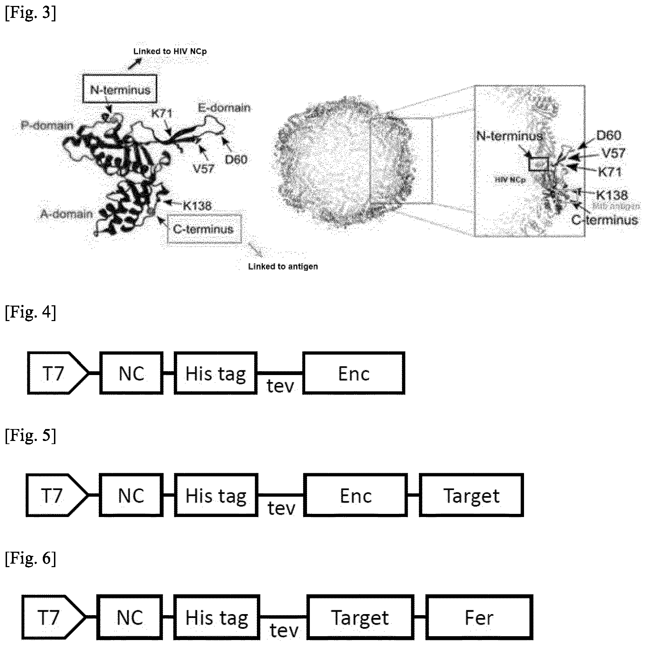

To investigate the applicability of the combination of a crystallizable protein and a viral nucleocapsid (NC) as fusion partners for producing self-assembled nanoparticles, a vector was manufactured to be expressed in such a form that NC of human immunodeficiency virus (HIV) was linked to the N-terminus of the crystallizable protein, encapsulin, and six histidine tags and a tobacco etch virus (TEV) protease cleavage site were present between them (FIG. 4).

The vector was manufactured based on a pGEMEX-1 (Promega) vector designed to regulate the expression of a target protein by a T7 promoter, and the sequence of SEQ ID NO: 5 was used as a sequence encoding encapsulin, and the sequence of SEQ ID NO: 2 (or SEQ ID NO: 3) was used as a sequence encoding NC.

The sequence of the construct region in the manufactured vector is the same as SEQ ID NO: 8, and the amino acid sequence of an expected expression fusion protein is the same as SEQ ID NO: 9.

### Example 1

A vector for producing self-assembled nanoparticles of a fusion protein was manufactured in a form in which NC was linked to the N-terminus of a crystallizable protein encapsulin, six histidine and TEV protease cleavage sites were present therebetween, and a target protein, which is a COVID19 receptor-binding domain (RBD), was linked to the C-terminus of the encapsulin (FIG. 5).

The manufacturing process was the same as in Preparation Example 1, but a sequence encoding the NC - 6X His tag - TEV protease cleavage site - encapsulin - target protein construct was inserted into the MCS of the pGEMEX -1(Promega) vector.

The sequence of the construct region in the manufactured vector is the same as SEQ ID NO: 10, and the amino acid sequence of an expected expression fusion protein is the same as SEQ ID NO: 11.

### Example 2

A vector for producing self-assembled nanoparticles of a fusion protein was manufactured in a form in which a target protein, influenza antigen (H5N1 HA: H5gd) was linked to the N-terminus of a crystallizable protein ferritin, NC was linked to the N-terminus of the influenza antigen, and six histidine tags and a TEV protease cleavage site were present between the NC and influenza antigen (FIG. 6).

The manufacturing process was the same as in Preparation Example 1, but a sequence encoding the NC - 6X His tag - TEV protease cleavage site - target protein - ferritin construct was inserted into the MCS of the pGEMEX-1 (Promega) vector.

The sequence of the construct region in the manufactured vector is the same as SEQ ID NO: 12, and the amino acid sequence of an expected expression fusion protein is the same as SEQ ID NO: 13.

### Experimental Example

The vectors of Preparation Example and Examples were transformed into *E*. *coli* SHuffle/T7(NEB) or SHuffle/T7/pLsS(NEB) and cultured. Shuffle/T7/pLysS was made by extracting a pLysS plasmid from BL21 star (DE3) pLysS One Shot^{™} competent *E. coli* (Invitrogen) using Mini-prep and introducing the extracted plasmid into Shuffle T7(NEB) competent *E*. *coli,* and selecting with chloramphenicol (CM), an antibiotic marker included in the pLysS. All transformed *E. coli* was cultured in LB media containing 50 µg/mL of ampicillin alone, or 50 µg/mL of ampicillin and 34 µg/mL of chloramphenicol. The culture temperature was set to 16 to 37 °C. When the OD600 value of *E. coli* became 0.5 or higher, IPTG was added at a level of 0 µM to 1 mM to activate the T7 promoter, and the culture was performed at 37 °C for 3 hours or at 25 °C for 5 hours to allow sufficient protein production after adding IPTG. Sufficiently cultured *E. coli* was centrifuged to remove the supernatant, which was then stored. Subsequently, 0.3 mL of PBS was added to the *E. coli* harvest, which corresponds to 5 mL of the LB medium, and subjected to sonication to obtain a lysate. Then, the lysate was centrifuged and divided into a soluble fraction and a pellet fraction, and the total lysate, the soluble fraction, and the pellet fraction were each analyzed by SDS-PAGE.

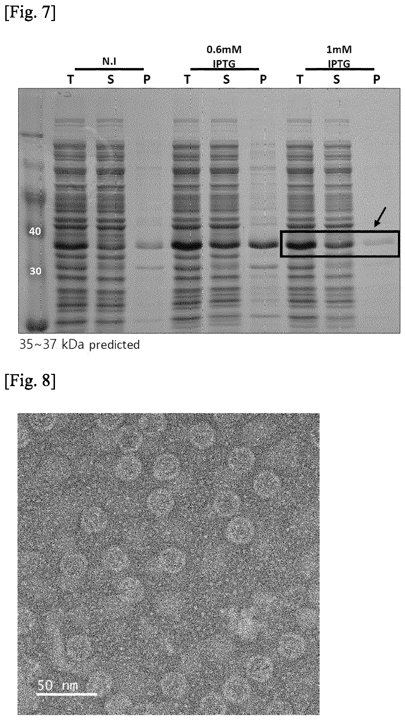

First, as a result of inducing the expression of a fusion protein by introducing the vector of Preparation Example 1 into *E. coli,* as shown in FIG. 7, a high level of the fusion protein was expressed normally, that is, with the expected size and solubility. This shows that the combination of the encapsulin protein and NC has the potential to be utilized as a fusion partner for producing target protein-fused self-assembled nanoparticles.

In addition, as a result of observing the product obtained by expression induction through transmission electron microscopy (TEM), as shown in FIG. 8, it was shown that the product has the form of nanoparticles.

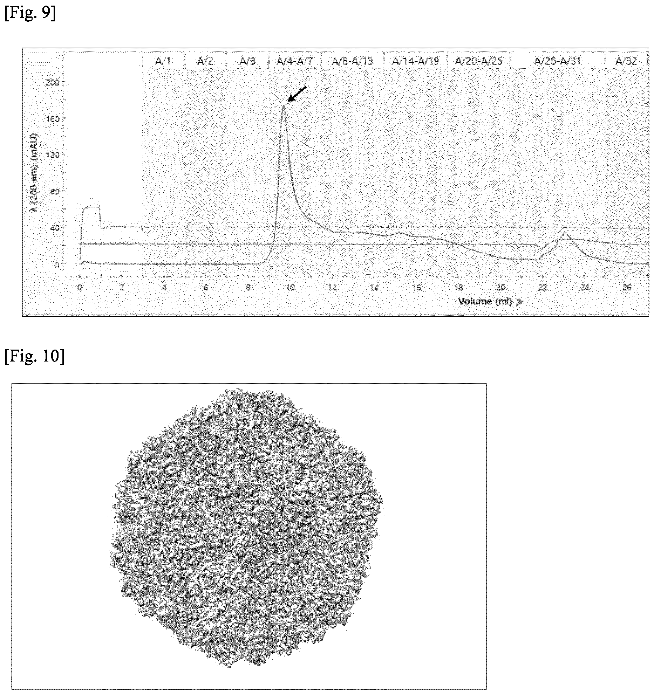

In addition, as a result of performing size-exclusion chromatography (SEC) on the product obtained by expression induction, as shown in FIG. 9, it was shown that the product has a constant size distribution.

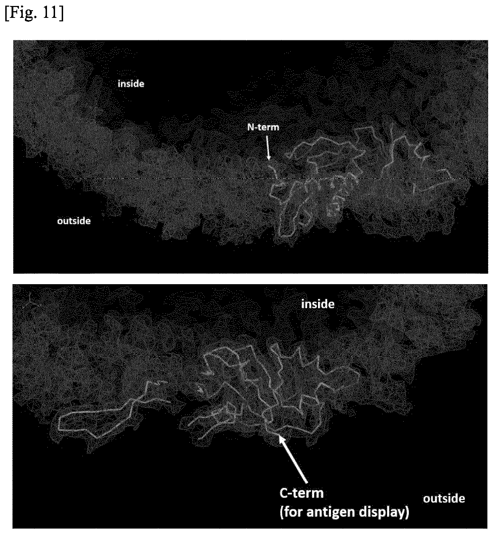

In addition, as a result of observing the product obtained by expression induction through Cryo-EM, as shown in FIG. 10, it was shown to have the form of spherical nanoparticles. Further, as a result of analyzing the positions of the N- and C-termini of the fusion protein after Cryo-EM, as shown in FIG. 11, it was shown that the N-terminus of the fusion protein is located inside the nanoparticle, and the C-terminus thereof is located outside the nanoparticle. These results demonstrated that when NC is linked to the N-terminus of the encapsulin and an antigen protein as a target protein is linked to the C-terminus, the antigen protein is exposed to the outside of the nanoparticle and acts as an antigen, and the NC is located inside the nanoparticle, thereby preventing unnecessary interference with an immune response, and thus highly efficient antigenic nanoparticles can be produced.

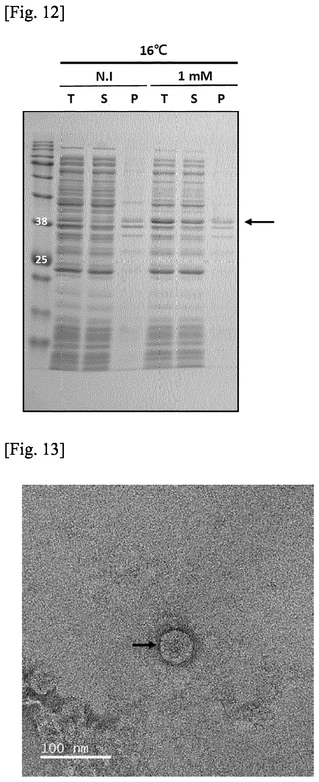

As a result of inducing the expression of a fusion protein by introducing the vector of Example 1 into *E. coli,* as shown in FIG. 12, , a high level of the fusion protein was expressed normally, that is, with the expected size and solubility.

In addition, as a result of observing the product obtained by inducing the expression of the vector of Example 1 through transmission electron microscopy (TEM), as shown in FIG. 13, it was shown that the product has the form of nanoparticles.

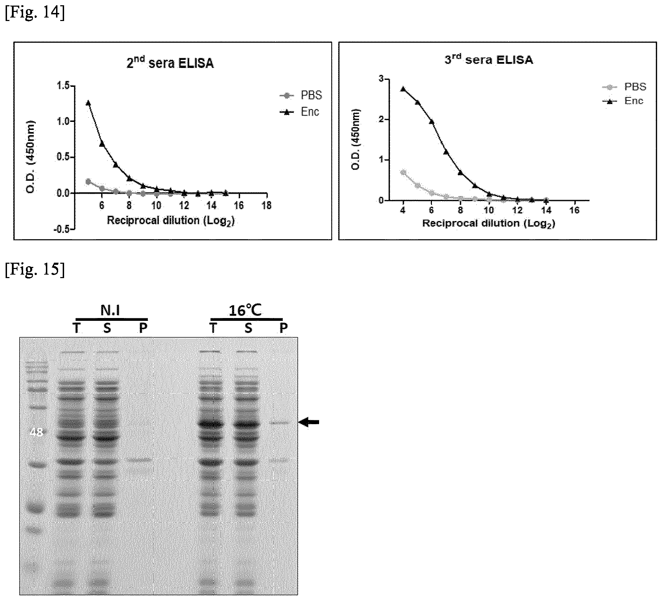

In addition, the product obtained by inducing the expression of the vector of Example 1, that is, the self-assembled nanoparticles were injected intraperitoneally (IP) or intramuscularly (IM) into mice at appropriate doses, and two weeks later, serum was obtained from orbital blood collection, and then ELISA was performed. The injection was performed three times at two-week intervals. As a result, as shown in FIG. 14, an IgG titer was shown. This demonstrates that, according to the method of the present invention, self-assembled nanoparticles in which the target protein is fused while maintaining its original structure can be produced.

As a result of inducing the expression of a fusion protein by introducing the vector of Example 2 into *E. coli,* as shown in FIG. 15, a high level of the fusion protein was expressed normally, that is, with the expected size and solubility.

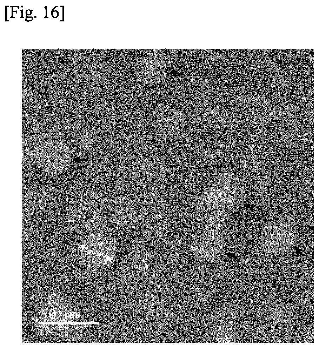

In addition, as a result of observing a product obtained by inducing the expression of the vector of Example 2 through transmission electron microscopy (TEM), as shown in FIG. 16, it was shown that the product has the form of nanoparticles.

In the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

### <Sequence listing>

SEQ ID NO: 1
   Sequence name : Amino acid sequence of HIV nucleocapsid
   Sequence type : AA
   Name of organism : human immunodeficiency virus
   Sequence : 
QRGNFRNQRKTVKCFNCGKEGHIAKNCRAPRKKGCWRCGREGHQM
KDCTERQAN
SEQ ID NO: 2
   Sequence name : Nucleotide sequence encoding HIV nucleocapsid
   Sequence type : DNA
   Name of organism : human immunodeficiency virus
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAAC
SEQ ID NO: 3
   Sequence name : Nucleotide sequence encoding HIV nucleocapsid
   Sequence type : DNA
   Name of organism : synthetic construct
   Sequence : 
CAGCGTGGTAACTTCCGTAACCAGCGTAAAACCGTTAAATGCTTCA
ACTGCGGCAAAGAAGGCCACATCGCGAAAAACTGCCGTGCGCCGCGTAA
 
AAAAGGCTGCTGGCGTTGCGGCCGTGAAGGCCACCAGATGAAAGATTGC
ACCGAACGTCAGGCGAAC
SEQ ID NO: 4
   Sequence name : Amino acid sequence of encapsulin from Mycobacterium tuberculosis
   Sequence type : AA
   Name of organism : Mycobacterium tuberculosis
   Sequence : 
NNLYRDLAPVTEAAWAEIELEAARTFKRHIAGRRVVDVSDPGGPVTA
AVSTGRLIDVKAPTNGVIAHLRASKPLVRLRVPFTLSRNEIDDVERGSKDSDW
EPVKEAAKKLAFVEDRTIFEGYSAASIEGIRSASSNPALTLPEDPREIPDVISQA
LSELRLAGVDGPYSVLLSADVYTKVSETSDHGYPIREHLNRLVDGDIIWAPAI
DGAFVLTTRGGDFDLQLGTDVAIGYASHDTDTVRLYLQETLTFLCYTAEASV
ALSH
SEQ ID NO: 5
   Sequence name : Nucleotide sequence encoding encapsulin from Mycobacterium tuberculosis
   Sequence type : DNA
   Name of organism : Mycobacterium tuberculosis
   Sequence : 
AATAACCTGTACCGGGACCTTGCACCAGTCACTGAGGCTGCGTGGG
CCGAGATCGAGCTTGAGGCCGCACGTACGTTTAAGCGTCATATTGCGGGC
CGTCGGGTTGTGGATGTATCAGATCCTGGAGGTCCTGTAACCGCTGCGGTA
TCGACAGGGCGTTTGATTGACGTAAAGGCTCCAACCAACGGTGTCATTGC
CCACTTACGCGCCAGCAAGCCTTTAGTTCGTTTAAGAGTGCCCTTTACGTT
AAGCCGGAACGAAATTGATGATGTGGAACGTGGGTCAAAGGACAGTGATT
GGGAACCAGTGAAAGAGGCGGCGAAGAAGTTGGCCTTCGTCGAAGATCG
TACCATATTTGAAGGCTACTCCGCTGCTTCCATAGAAGGGATTCGGTCCGC
CTCCTCAAATCCAGCATTAACGCTGCCTGAAGACCCGCGCGAGATTCCTG
 
ATGTAATCTCACAGGCACTTTCGGAATTACGTTTGGCAGGGGTCGATGGAC
CATACAGTGTTCTTTTGAGTGCAGATGTATACACCAAGGTGAGCGAGACC
AGTGATCACGGCTATCCAATACGGGAACACCTGAACAGACTTGTCGATGG
CGACATAATCTGGGCGCCCGCAATTGACGGCGCATTCGTGTTGACCACCCG
TGGTGGTGATTTTGATTTACAATTAGGCACCGATGTTGCGATTGGGTATGCG
AGTCATGATACGGACACTGTACGTTTGTATCTTCAAGAGACGCTGACGTTC
TTATGTTACACCGCTGAGGCGTCCGTGGCGCTTTCGCAT
SEQ ID NO: 6
   Sequence name : Amino acid sequence of ferritin from Escherichia coli
   Sequence type : AA
   Name of organism : Escherichia coli
   Sequence : 
KGDTKVINYLNKLLGNELVAINQYFLHARMFKNWGLKRLNDVEYHE
SIDEMKHADRYIERILFLEGLPNLQDLGKLNIGEDVEEMLRSDLALELDGAK
NLREAIGYADSVHDYVSRDMMIEILRDEEGHIDWLETELDLIQKMGLQNYLQ
AQIREEG
SEQ ID NO: 7
   Sequence name : Nucleotide sequence encoding ferritin from Escherichia coli
   Sequence type : DNA
   Name of organism : Escherichia coli
   Sequence : 
AAAGGTGATACTAAAGTTATAAATTATCTCAACAAACTGTTGGGAA
ATGAGCTTGTCGCAATCAATCAGTACTTTCTCCATGCCCGAATGTTTAAAA
ACTGGGGTCTCAAACGTCTCAATGATGTGGAGTATCATGAATCCATTGATG
AGATGAAACACGCCGATCGTTATATTGAGCGCATTCTTTTTCTGGAAGGTC
TTCCAAACTTACAGGACCTGGGCAAACTGAACATTGGTGAAGATGTTGAG
GAAATGCTGCGTTCTGATCTGGCACTTGAGCTGGATGGCGCGAAGAATTT
GCGTGAGGCAATTGGTTATGCCGATAGCGTTCATGATTACGTCAGCCGCGA
TATGATGATAGAAATTTTGCGTGATGAAGAAGGCCATATCGACTGGCTGGA
 
AACGGAACTTGATCTGATTCAGAAGATGGGCCTGCAAAATTATCTGCAAG
CACAGATCCGCGAAGAAGGT
SEQ ID NO: 8
   Sequence name : Nucleotide sequence of fusion protein construct
   Sequence type : DNA
   Name of organism : synthetic construct
   Sequence : 
ATGCAGCGGGGAAACTTCAGGAACCAGAGAAAAACTGTGAAGTG
CTTCAATTGCGGAAAGGAGGGCCACATCGCTAAGAACTGCCGCGCCCCCA
GAAAGAAAGGCTGCTGGAGATGCGGCAGAGAGGGCCACCAGATGAAGG
ACTGCACAGAGAGACAGGCAAACCATCACCATCACCATCACGAAAACCT
GTATTTTCAGGGTGGATCCAATAACCTGTACCGGGACCTTGCACCAGTCAC
TGAGGCTGCGTGGGCCGAGATCGAGCTTGAGGCCGCACGTACGTTTAAGC
GTCATATTGCGGGCCGTCGGGTTGTGGATGTATCAGATCCTGGAGGTCCTG
TAACCGCTGCGGTATCGACAGGGCGTTTGATTGACGTAAAGGCTCCAACC
AACGGTGTCATTGCCCACTTACGCGCCAGCAAGCCTTTAGTTCGTTTAAGA
GTGCCCTTTACGTTAAGCCGGAACGAAATTGATGATGTGGAACGTGGGTC
AAAGGACAGTGATTGGGAACCAGTGAAAGAGGCGGCGAAGAAGTTGGCC
TTCGTCGAAGATCGTACCATATTTGAAGGCTACTCCGCTGCTTCCATAGAA
GGGATTCGGTCCGCCTCCTCAAATCCAGCATTAACGCTGCCTGAAGACCC
GCGCGAGATTCCTGATGTAATCTCACAGGCACTTTCGGAATTACGTTTGGC
AGGGGTCGATGGACCATACAGTGTTCTTTTGAGTGCAGATGTATACACCAA
GGTGAGCGAGACCAGTGATCACGGCTATCCAATACGGGAACACCTGAACA
GACTTGTCGATGGCGACATAATCTGGGCGCCCGCAATTGACGGCGCATTCG
TGTTGACCACCCGTGGTGGTGATTTTGATTTACAATTAGGCACCGATGTTG
CGATTGGGTATGCGAGTCATGATACGGACACTGTACGTTTGTATCTTCAAG
AGACGCTGACGTTCTTATGTTACACCGCTGAGGCGTCCGTGGCGCTTTCGC
ATGATATCGTCGACAAGCTTTGA
SEQ ID NO: 9
   Sequence name : Amino acid sequence of expected fusion protein
   Sequence type : AA
   Name of organism : synthetic construct
   Sequence : 
MQRGNFRNQRKTVKCFNCGKEGHIAKNCRAPRKKGCWRCGREGHQ
MKDCTERQANHHHHHHENLYFQGGSNNLYRDLAPVTEAAWAEIELEAARTF
KRHIAGRRVVDVSDPGGPVTAAVSTGRLIDVKAPTNGVIAHLRASKPLVRLR
VPFTLSRNEIDDVERGSKDSDWEPVKEAAKKLAFVEDRTIFEGYSAASIEGIR
SASSNPALTLPEDPREIPDVISQALSELRLAGVDGPYSVLLSADVYTKVSETSD
HGYPIREHLNRLVDGDIIWAPAIDGAFVLTTRGGDFDLQLGTDVAIGYASHDT
DTVRLYLQETLTFLCYTAEASVALSHDIVDKL
SEQ ID NO: 10
   Sequence name : Nucleotide sequence of fusion protein construct
   Sequence type : DNA
   Name of organism : synthetic construct
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAACGAAAACCTGTATTTTCAGGGCCATCACCATC
ACCATCACGATGATGATGATGGTACCGGATCCAATAACCTGTACCGGGACC
TTGCACCAGTCACTGAGGCTGCGTGGGCCGAGATCGAGCTTGAGGCCGCA
CGTACGTTTAAGCGTCATATTGCGGGCCGTCGGGTTGTGGATGTATCAGAT
CCTGGAGGTCCTGTAACCGCTGCGGTATCGACAGGGCGTTTGATTGACGT
AAAGGCTCCAACCAACGGTGTCATTGCCCACTTACGCGCCAGCAAGCCTT
TAGTTCGTTTAAGAGTGCCCTTTACGTTAAGCCGGAACGAAATTGATGATG
TGGAACGTGGGTCAAAGGACAGTGATTGGGAACCAGTGAAAGAGGCGGC
GAAGAAGTTGGCCTTCGTCGAAGATCGTACCATATTTGAAGGCTACTCCGC
TGCTTCCATAGAAGGGATTCGGTCCGCCTCCTCAAATCCAGCATTAACGCT
 
GCCTGAAGACCCGCGCGAGATTCCTGATGTAATCTCACAGGCACTTTCGG
AATTACGTTTGGCAGGGGTCGATGGACCATACAGTGTTCTTTTGAGTGCAG
ATGTATACACCAAGGTGAGCGAGACCAGTGATCACGGCTATCCAATACGG
GAACACCTGAACAGACTTGTCGATGGCGACATAATCTGGGCGCCCGCAAT
TGACGGCGCATTCGTGTTGACCACCCGTGGTGGTGATTTTGATTTACAATT
AGGCACCGATGTTGCGATTGGGTATGCGAGTCATGATACGGACACTGTACG
TTTGTATCTTCAAGAGACGCTGACGTTCTTATGTTACACCGCTGAGGCGTC
CGTGGCGCTTTCGCATGATATCGTCGACAACATAACAAATCTATGTCCCTTT
GGAGAAGTGTTTAACGCGACCCGTTTTGCCAGCGTTTACGCATGGAATCG
CAAGCGCATTTCTAACTGCGTGGCGGACTACAGCGTGCTGTATAATTCCGC
GTCGTTCTCTACGTTTAAATGCTACGGCGTGAGCCCGACTAAGTTGAATGA
TCTGTGTTTTACCAATGTTTACGCTGACAGCTTTGTTATTCGTGGTGATGAG
GTTCGTCAGATTGCGCCAGGTCAGACCGGTAAAATCGCGGACTATAACTAC
AAACTTCCGGATGATTTCACCGGCTGCGTCATCGCCTGGAACTCCAACAA
CTTGGACTCGAAGGTGGGTGGTAATTACAACTACCTCAGACGTCTGTTCC
GCAAGTCTAACCTGAAACCGTTTGAACGTGACATCAGCACCGAGATCTAC
CAAGCGGGTAGCACCCCGTGTAATGGCGTTGAAGGTTTCAACTGCTATTTC
CCGCTGCAGAGCTATGGCTTCCAACCGACGAACGGCGTGGGCTATCAACC
GTATCGTGTTGTCGTACTGAGCTTCGAGAACAAGTCCGCTCCGGCAACCG
TTTGCGGTCCG
SEQ ID NO: 11
   Sequence name : Amino acid sequence of expected fusion protein
   Sequence type : AA
   Name of organism : synthetic construct
   Sequence : 
QRGNFRNQRKTVKCFNCGKEGHIAKNCRAPRKKGCWRCGREGHQM
KDCTERQANENLYFQGHHHHHHDDDDGTGSNNLYRDLAPVTEAAWAEIELE
AARTFKRHIAGRRVVDVSDPGGPVTAAVSTGRLIDVKAPTNGVIAHLRASKP
LVRLRVPFTLSRNEIDDVERGSKDSDWEPVKEAAKKLAFVEDRTIFEGYSAAS
 
IEGIRSASSNPALTLPEDPREIPDVISQALSELRLAGVDGPYSVLLSADVYTKVS
ETSDHGYPIREHLNRLVDGDIIWAPAIDGAFVLTTRGGDFDLQLGTDVAIGYA
SHDTDTVRLYLQETLTFLCYTAEASVALSHDIVDNITNLCPFGEVFNATRFASV
YAWNRKRISNCVADYSVLYNSASFSTFKCYGVSPTKLNDLCFTNVYADSFVIR
GDEVRQIAPGQTGKIADYNYKLPDDFTGCVIAWNSNNLDSKVGGNYNYLRR
LFRKSNLKPFERDISTEIYQAGSTPCNGVEGFNCYFPLQSYGFQPTNGVGYQP
YRVVVLSFENKSAPATVCGP
SEQ ID NO: 12
   Sequence name : Nucleotide sequence of fusion protein construct
   Sequence type : DNA
   Name of organism : synthetic construct
   Sequence : 
CAGCGGGGAAACTTCAGGAACCAGAGAAAAACTGTGAAGTGCTTC
AATTGCGGAAAGGAGGGCCACATCGCTAAGAACTGCCGCGCCCCCAGAA
AGAAAGGCTGCTGGAGATGCGGCAGAGAGGGCCACCAGATGAAGGACTG
CACAGAGAGACAGGCAAACCATCATCATCATCATCATGAAAACCTGTATTT
TCAGGGTGGTACCGGATCCGGAGTGAAGCCTCTAATTTTAAGAGATTGTAG
TGTAGCTGGATGGCTCCTCGGGAACCCAATGTGTGACGAATTCATCAATGT
ACCGGAATGGTCTTACATAGTGGAGAAGGCCAATCCAACCAATGACCTCT
GTTACCCAGGGAGTTTCAACGACTATGAAGAACTGAAACACCTATTGAGC
AGAATAAACCATTTTGAGAAAATTCAAATCATCCCCAAAAGTTCTTGGTCC
GATCATGAAGCCTCATCAGGAGTGAGCTCAGCATGTCCATACCTGGGAAG
TCCCTCCTTTTTTAGAAATGTGGTATGGCTTATCAAAAAGAACAGTACATA
CCCAACAATAAAGAAAAGCTACAATAATACCAACCAAGAAGATCTTTTGG
TACTGTGGGGAATTCACCATCCTAATGATGCGGCAGAGCAGACAAGGCTAT
ATCAAAACCCAACCACCTATATTTCCATTGGGACATCAACACTAAACCAGA
GATTGGTACCAAAAATAGCTACTAGATCCAAAGTAAACGGGCAAAGTGGA
AGGATGGAGTTCTTCTGGACAATTTTAAAACCTAATGATGCAATCAACTTC
GAGAGTAATGGAAATTTCATTGCTCCAGAATATGCATACAAAATTGTCAAG
 
AAAGGGGACTCAGCAATTATGAAAAGTGAATTGGATATCGTCGACATGAA
AGGTGATACTAAAGTTATAAATTATCTCAACAAACTGTTGGGAAATGAGCT
TGTCGCAATCAATCAGTACTTTCTCCATGCCCGAATGTTTAAAAACTGGGG
TCTCAAACGTCTCAATGATGTGGAGTATCATGAATCCATTGATGAGATGAA
ACACGCCGATCGTTATATTGAGCGCATTCTTTTTCTGGAAGGTCTTCCAAA
CTTACAGGACCTGGGCAAACTGAACATTGGTGAAGATGTTGAGGAAATGC
TGCGTTCTGATCTGGCACTTGAGCTGGATGGCGCGAAGAATTTGCGTGAG
GCAATTGGTTATGCCGATAGCGTTCATGATTACGTCAGCCGCGATATGATGA
TAGAAATTTTGCGTGATGAAGAAGGCCATATCGACTGGCTGGAAACGGAA
CTTGATCTGATTCAGAAGATGGGCCTGCAAAATTATCTGCAAGCACAGATC
CGCGAAGAAGGT
SEQ ID NO: 13
   Sequence name : Amino acid sequence of expected fusion protein
   Sequence type : AA
   Name of organism : synthetic construct
   Sequence :

QRGNFRNQRKTVKCFNCGKEGHIAKNCRAPRKKGCWRCGREGHQM
KDCTERQANHHHHHHENLYFQGGTGSGVKPLILRDCSVAGWLLGNPMCDEF
INVPEWSYIVEKANPTNDLCYPGSFNDYEELKHLLSRINHFEKIQIIPKSSWSD
HEASSGVSSACPYLGSPSFFRNVVWLIKKNSTYPTIKKSYNNTNQEDLLVLW
GIHHPNDAAEQTRLYQNPTTYISIGTSTLNQRLVPKIATRSKVNGQSGRMEFF
WTILKPNDAINFESNGNFIAPEYAYKIVKKGDSAIMKSELDIVDMKGDTKVIN
YLNKLLGNELVAINQYFLHARMFKNWGLKRLNDVEYHESIDEMKHADRYIE
RILFLEGLPNLQDLGKLNIGEDVEEMLRSDLALELDGAKNLREAIGYADSVH
DYVSRDMMIEILRDEEGHIDWLETELDLIQKMGLQNYLQAQIREEG

## Claims

1. An expression vector for producing target protein-fused self-assembled nanoparticles, comprising a polynucleotide that encodes a crystallizable protein and a viral nucleocapsid as fusion partners of a target protein.

2. The expression vector of claim 1, wherein the target protein is at least one selected from the group consisting of an antigen, an antibody, a cell receptor, an enzyme, a structural protein, serum, and a cell protein.

3. The expression vector of claim 1, wherein the crystallizable protein is encapsulin or ferritin.

4. The expression vector of claim 1, wherein the viral nucleocapsid is a nucleocapsid of human immunodeficiency virus (HIV).

5. The expression vector of claim 1, wherein the viral nucleocapsid includes the amino acid sequence of SEQ ID NO: 1.

6. The expression vector of claim 1, wherein the vector is for producing target protein-fused self-assembled nanoparticles *in E. coli.*

7. The expression vector of claim 1, wherein the polynucleotide encodes a crystallizable protein and a viral nucleocapsid linked to the N-terminus of the crystallizable protein, and includes a cloning site for linking a target protein-coding site or target protein to the C-terminus of the crystallizable protein.

8. A host cell which is transformed with the expression vector for producing target protein-fused self-assembled nanoparticles of any one of claims 1 to 7.

9. A method of producing target protein-fused self-assembled nanoparticles, comprising:
manufacturing an expression vector for producing target protein-fused self-assembled nanoparticles, including a polynucleotide that encodes a target protein and a crystallizable protein and a viral nucleocapsid as fusion partners of the target protein;
producing a transformant by introducing the expression vector for producing target protein-fused self-assembled nanoparticles into host cells; and
culturing the transformant.

10. The method of claim 9, wherein the target protein is at least one selected from the group consisting of an antigen, an antibody, a cell receptor, an enzyme, a structural protein, a serum, and a cell protein.

11. The method of claim 9, wherein the crystallizable protein is encapsulin or ferritin.

12. The method of claim 9, wherein the viral nucleocapsid is a nucleocapsid of human immunodeficiency virus (HIV).

13. The method of claim 9, wherein the viral nucleocapsid includes the amino acid sequence of SEQ ID NO: 1.

14. The method of claim 9, wherein the host cell is *E. coli.*

15. The method of claim 9, wherein the polynucleotide encodes the crystallizable protein, a viral nucleocapsid linked to the N-terminus of the crystallizable protein, and a target protein linked to the C-terminus of the crystallizable protein.
